# EUROPÄISCHE PATENTANMELDUNG

(11) **EP 3 995 201 A1**
(43) Veröffentlichungstag der Anmeldung: **11.05.2022**
(21) Anmeldenummer: 21186837.7
(22) Anmeldetag: 21.07.2021
(51) Int. Cl.: B01D 46/12

(54) **VORRICHTUNG ZUR FILTRATION VON LUFT**

(30) Priorität: 06.11.2020 DE 202020106370 U
(71) Anmelder: Krantz GmbH, 52072 Aachen (DE)
(72) Erfinder: Felser, Hans, 52080 Aachen (DE); Sauder, Marco, 52249 Eschweiler (DE); Schröder, Norbert, 46569 Hünxe (DE)
(74) Vertreter: Bauer, Dirk

(57) **Zusammenfassung**

Eine Vorrichtung (1) zur Filtration von Luft umfasst
- ein einen Innenraum (2) umschließendes Gehäuse (3) mit einem Lufteintrittsquerschnitt (4) und einem Luftaustrittsquerschnitt (5),
- einer Vorfilterstufe (9) mit einem ersten Filterelement (10) mit einem ersten Filtermedium (11),
- eine Hauptfilterstufe (6) mit einem zweiten Filterelement (7) mit einem zweiten Filtermedium (8),
- eine Luftfördereinrichtung (12), mit der die Luft von dem Lufteintrittsquerschnitt (4) durch die Vorfilterstufe (9) und die Hauptfilterstufe (6) zu dem Luftaustrittsquerschnitt (5) förderbar ist,
wobei das zweite Filterelement (7) den Innenraum (2) in einen Rohluftraum (13), in dem sich die Vorfilterstufe (9) befindet, und einen Reinluftraum (14), in dem sich die Luftfördereinrichtung (12) befindet, unterteilt. Um eine sehr wirksame, aber gleichfalls mit einem geringen Energiebedarf verbundene Desinfektion zu erreichen wir vorgeschlagen, dass mit Luft in Berührung kommende Oberflächen, die den Rohluftraum (13) begrenzen, mit einer antimikrobiell wirksamen, insbesondere Viren inaktivierenden, Substanz versehen sind.

## Beschreibung

Die Erfindung betrifft eine Vorrichtung zur Filtration von Luft.

Die Vorrichtung umfasst ein Gehäuse, welches beispielsweise eine Quaderform aufweist, mit zugehörigem Innenraum. Das Gehäuse wird an seiner Oberseite durch einen Luftaustrittsquerschnitt und an einer Unterseite durch einen Lufteintrittsquerschnitt begrenzt.

Im Innenraum des Gehäuses zwischen Lufteintrittsquerschnitt und Luftaustrittsquerschnitt befinden sich eine Vorfilterstufe, eine Hauptfilterstufe und sowie eine Luftfördereinrichtung. Die Vorfilterstufe besteht aus einem ersten Filterelement sowie einem ersten Filtermedium. Die Hauptfilterstufe umfasst ein zweites Filterelement mit dazugehörigem zweiten Filtermedium. Die zu filternde Luft wird mittels der Luftfördereinrichtung von dem Lufteintrittsquerschnitt durch die Vorfilterstufe und die Hauptfilterstufe zu dem Luftaustrittsquerschnitt gefördert. Das zweite Filterelement teilt den Innenraum des Gehäuses in einen Rohluftraum, in dem sich die Vorfilterstufe befindet, und einen Reinluftraum, in dem sich die Luftfördereinrichtung befindet.

### Stand der Technik

Vorrichtungen gemäß der oben genannten Art weisen z.B. keine Inaktivierung der Mikroorganismen auf, wodurch bei dem Wechsel der Filterelemente die Gefahr besteht, dass das ausführende Personal und die Umgebung kontaminiert werden. Die vorgenannten Vorrichtungen ohne Inaktivierung führen bei einem eventuellen Filterbruch und Weiterbetrieb zu einer gefährlichen Verteilung der Mikroorganismen im Raum und somit zu einer erhöhten Gefährdung im Raum befindlicher Personen. Ferner finden in den Filterstufen oben genannter Vorrichtungen unkontrollierte Vorgänge, z.B. eine nicht kontrollierbare Vermehrung, statt, sollte es sich bei der Kontamination um lebende Mikroorganismen handeln und ein aus anderen Filterrückständen gebildeter Nährboden vorhanden sein.

Bei allgemein bekannten Vorrichtungen mit Inaktivierung von Mikroorganismen erfolgt die Inaktivierung entweder über das periodische Erhitzen der Filterstufen oder durch die Bestrahlung mit UVB-C Licht. Erstes Verfahren ist nachteilig, insofern es einen hohen Energieverbrauch aufweist, um die zum Inaktivieren nötige Temperatur zu erreichen. Außerdem führt das Verfahren zu einer zusätzlichen Erwärmung der Raumluft auch zu Zeiten, in denen eine Erwärmung nicht gewünscht ist, wie zum Beispiel im Sommer. Das letztere Verfahren hat den Nachteil, dass die Verweilzeit der Mikroorganismen eingehalten werden muss, um die Inaktivierung zu gewährleisten. Dies führt zu einem geringeren förderbaren Volumenstrom.

Ferner besteht bei letzterem Verfahren die Gefahr der unerwünschten Ozon-Bildung. Darüber hinaus weisen beide Verfahren den Nachteil auf, insofern beide Vorrichtungen eine gesonderte Energiezufuhr benötigen, um das Erhitzen oder UVB-C Licht zu ermöglichen. Dies führt zu dem Risiko, dass ein Defekt von Komponenten, die zum Inaktivieren notwendig sind, unentdeckt bleibt.

Aus der DE 602 23 231 T2 ist ein Wärmetauscher und ein mit diesem ausgestattetes Klimagerät bekannt, bei dem eine einfachere Reinigung oder Sterilisierung gegen LegionellaBakterien möglich ist. Neben einer besonderen sinus-förmigen Profilform bei den verwendeten Rippen-Rohr-Elementen schlägt die DE 602 23 231 T2 vor, eine äußere Oberfläche der Rippen-Rohr-Elemente mit einem antimikrobiellen Mittel zu beschichten, wobei es sich bei der Beschichtung um ein Silan handelt, das mit einem quaternären Amin versehen ist. Die mit dem vorgenannten antimikrobiellen Mittel beschichteten Oberflächen sollen zusätzlich in einer UV-Luftreinigungsstation mit kurzwelligem UV-Licht im Bereich zwischen 184 nm bis 254 nm Wellenlänge bestrahlt werden. Hierdurch wird wiederum der Energieverbrauch des bekannten Wärmetauschers erhöht.

### Aufgabe

Der Erfindung liegt mithin die Aufgabe zugrunde, eine Vorrichtung zur verbesserten Filtration der Luft bereit zu stellen, die sich durch ein sehr sicheres Inaktivieren in der Luft vorhandener Mikroorganismen auszeichnet und keine Hilfsenergie zur Inaktivierung bedarf.

### Lösung

Die vorstehende Aufgabe wird, ausgehend von einem Luftauslass der eingangs beschriebenen Art, erfindungsgemäß dadurch gelöst, dass mit Luft in Berührung kommende Oberflächen, die den Rohluftraum begrenzen, mit einer antimikrobiell wirksamen , insbesondere Viren inaktivierenden, Substanz versehen sind. Vorteilhafte Ausführungen ergeben sich aus den Merkmalen der Unteransprüche.

Ein besonderer Vorteil der erfindungsgemäßen Vorrichtung besteht darin, dass die Inaktivierung der Mikroorganismen durch die vorhandene antimikrobiell wirkende Substanz erfolgt. Die Mikroorganismen werden bei Kontakt mit der antimikrobiell wirkenden Substanz an dieser gebunden und von dieser inaktiviert. Insbesondere wird zum Inaktivieren der Mikroorganismen keine zusätzliche Energiezufuhr benötigt, dies verhindert einerseits das Aufheizen des Raumes. Ferner ist das Risiko eines Defekts von Komponenten, die zum Inaktivieren notwendig sind, deutlich geringer. Gleichzeitig ist die Güte der Luftfiltration nicht an die Verweilzeit der Luft im Rohluftraum gekoppelt, sodass ein höherer Volumenstrom gefördert werden kann. Der förderbare Volumenstrom ist insbesondere ein wichtiges Kriterium für die Anzahl an Personen, die sich in einem Raum, in dem eine Vorrichtung zur Luftfiltration steht, aufhalten dürfen. Durch das räumliche Zusammenfallen von Zurückhalten (Festlegen) der Mikroorganismen an den Oberflächen im Rohluftraum einerseits und deren Inaktivierung mittels der eben dort vorhandenen inaktivierenden Substanz andererseits wird eine große Effektivität und Sicherheit der erfindungsgemäßen Vorrichtung erzielt.

Vorzugsweise wird eine Beschichtung entsprechend der WO2020/079233A2 verwendet. Eine solche Beschichtung weist verschiedene Polymerstrukturen auf, wobei mindestens ein Polymer Mikroorgansimen bindet und inaktiviert.

Eine vorteilhafte Ausgestaltung besteht in einem Frischluft- oder Außenluftanschluss und einer in dem Reinluftraum oder den Rohluftraum angeordneten Mischkammer, in der Frisch- oder Außenluft mit der gefilterten Luft mischbar und sodann vorzugsweise mittels derselben Luftfördereinrichtung zu dem Luftaustrittsquerschnitt förderbar ist. Hierdurch wird erreicht, dass zusätzliche Frisch- oder Außenluft, die eine geringere Konzentration an Mikroorganismen und einen gegenüber Umluft höheren Sauerstoffgehalt aufweist, in den Raum, in dem sich die Vorrichtung zur Filtration befindet, gefördert wird. Die Mischung der Frisch- oder Außenluft mit der Raumluft kann entweder im Rohluftraum oder im Reinluftraum erfolgen. Die Mischung im Rohluftraum hat den Vorteil, dass die Frisch- oder Außenluft zusammen mit der Raumluft gefiltert wird, bevor diese dem Raum zugeführt wird. Um die Wartungszyklen der Filterstufen geringer zu halten, kann es vorteilhaft sein, die Frisch- oder Außenluft erst im Reinluftraum mit der vorab gefilterten Luft zu vermischen.

Eine Weiterbildung der Erfindung besteht darin, dass der Innenraum des Gehäuses mit schalldämmenden Material, insbesondere einem Schaummaterial oder einem aus gepressten Fasern bestehenden Material, versehen, insbesondere damit isoliert ist, wobei das im Rohluftraum angeordnete schalldämmende Material zusätzlich mit einer antimikrobiell wirkenden Substanz versehen, insbesondere damit beschichtet, ist, und zwar insbesondere auf seiner dem Innenraum zugewandten Seite.

Insbesondere wenn sich Personen im Raum aufhalten, ist eine kontinuierliche Filterung von Luft notwendig. In Folge dessen ergibt sich die Notwendigkeit eines geräuscharmen Betriebs der Vorrichtung. Die Verkleidung des Innenraums mit schalldämmendem Material hat den Vorteil, dass die Schallemission reduziert wird. Die zur Schallisolierung verwendeten Materialien, insbesondere ein Schaummaterial oder gepresste Fasern, weisen oft eine poröse Oberflächenstruktur auf. Eine Beschichtung solcher Oberflächen ist insbesondere vorteilhaft, da die sich darin bzw. daran sammelnden Mikroorganismen bei Kontakt inaktiviert werden und anschließend bei z.B. Wartungsarbeiten keine Kontaminationsgefahr darstellen.

Es ist möglich, dass das akustische Dämmeigenschaften aufweisende Material plattenförmig ist und allein klemmend in dem Rohluftraum in seiner eingebauten Position gehalten ist. Hierdurch wird eine werkzeuglose Montage und Entnahme zu Wartungszwecken ermöglicht.

Vorzugsweise ist das erste Filtermedium und/oder das zweite Filtermedium durch Tauchen und/oder Besprühen und/oder sonstiges Auftragen eines fließfähigen Stoffes mit einer antibakteriell wirksamen Beschichtung versehen. Hierdurch wird erreicht, dass die antimikrobiell wirksame Substanz insbesondere auch auf solche Oberflächen, die in einer tieferen räumlichen Ebene des ersten und/ oder zweiten Filtermediums liegen, aufgetragen wird. Insbesondere kann somit mehr, insbesondere die komplette Fläche des ersten und/ oder zweiten Filtermediums beschichtet werden, wodurch eine sicherere Inaktivierung der zurückgehaltenen Mikroorganismen erreicht wird.

Die Erfindung weiter ausgestaltend können die Wandungen des zweiten Filterelements und/oder ersten Filterelements mit einer antimikrobiell wirksamen Substanz versehen werden. Dadurch werden Mikroorganismen, die mit den Wandungen des ersten Filterelements und/ oder zweiten Filterelements in Kontakt kommen und dort anhaften, inaktiviert. Dies ist insbesondere bei Wartungsarbeiten z.B. im Zuge eines Filterwechsels vorteilhaft, da hiermit das Risiko einer Kontamination des durchführenden Personals und der Umgebung reduziert wird.

Es ist möglich, dass der Lufteintrittsquerschnitt von Durchbrüchen eines Lochblechs gebildet wird, wobei eine dem Rohluftraum zugewandte Innenseite des Lochblechs mit einer antimikrobiell wirksamen Beschichtung versehen ist. Hierdurch wird eine zusätzlich Inaktivierung von in Ablagerungen auf besagtem Lochblech enthaltenen Mikroorganismen erreicht. Außerdem sinkt die sich im Rohluftraum zu dem Zeitpunkt des Anhaltens der Luftförderung durch die Luftfördereinrichtung befindliche Luft in Richtung Fußboden ab. Damit kommt die absinkende Luft zumindest teilweise in Kontakt mit der Innenseite des Lochblechs, wodurch Mikroorganismen inaktiviert werden. Dies ist besonders vorteilhaft bei der Wartung der Vorrichtung, da die Gefahr der Kontamination des Personals reduziert ist.

Eine vorteilhafte Ausgestaltung besteht darin, dass das Gehäuse ungefähr quaderförmig und zur Aufstellung auf einem Fußboden vorgesehen ist, wobei eine parallel zu dem Fußboden verlaufende Unterseite des Gehäuses den Lufteintrittsquerschnitt aufweist und eine parallel zu dem Fußboden verlaufende und dem Lufteintrittsquerschnitt gegenüberliegende Oberseite des Gehäuses den Luftaustrittsquerschnitt aufweist. Hierdurch wird eine vorteilhafte Luftzirkulation im zu filternden Raum erreicht. Insbesondere kann die gefilterte Luft aus dem Reinluftraum möglichst ungestört (typischerweise oberhalb von Möbeln) in den Raum strömen, sodass ein möglichst großes Raumvolumen von der ausströmenden Luft erfasst wird. Ferner werden Verschmutzungen, die sich auf dem Fußboden befinden, von der Hauptfilterstufe und Vorfilterstufe herausgefiltert, falls sie in die Vorrichtung eingesaugt werden.

Eine Weiterbildung der Erfindung besteht darin, dass eine Oberseite des Gehäuses und/ oder mindestens ein oberer Abschnitt mindestens einer seitlichen Wandung mit mindestens einem Luftdurchlass, insbesondere einem Drall-Luftdurchlass und/ oder einem Luftverteilelement, versehen ist. Weitere mögliche Ausführungsmöglichkeiten sind ein Fächerauslass, eine Düse, ein induktiver Auslass, ein Quellluftauslass und/ oder deren Kombination. Insbesondere ist ein zusätzlicher oder alleiniger im oberen Abschnitt einer seitlichen Wandung angeordneter Luftdurchlass mit individuell verstellbarer Richtung der Austrittsströmung vorteilhaft, da hierdurch eine Anpassung der Strömungscharakteristik z.B. in Abhängigkeit vom Aufstellungsort der Vorrichtung im Raum und eine Umwälzung und Filtrierung der gesamten Raumluft möglich ist. Ferner kann somit eine Kurzschlussströmung verhindert werden.

Eine vorteilhafte Ausgestaltung ist eine Differenzdruck-Messeinrichtung, mittels der ein Differenzdruck zwischen einer Eintrittsseite der Hauptfilterstufe oder der Vorfilterstufe und einer zugeordneten Austrittsseite der jeweiligen Filterstufe messbar ist. Es ist davon auszugehen, dass ein zunehmender Differenzdruck zwischen einer Eintrittsseite und einer zugehöriger Austrittsseite der Hauptfilterstufe und/ oder Vorfilterstufe darauf hinweist, dass die Hauptfilterstufe und/ oder Vorfilterstufe zunehmend beladen ist. Das heißt, insbesondere, dass der Durchströmungswiderstand zunimmt und der erreichbare Volumenstrom der Luft abnimmt und in Folge dessen ein Filterwechsel notwendig ist. Der Vorteil der Differenzdruck-Messeinrichtung besteht also insbesondere darin, dass damit ein frühzeitiger Hinweis, vorzugsweise in Form einer Visualisierung, auf den bevorstehenden Filterelementwechsel in der Hauptfilterstufe und/oder Vorfilterstufe ermöglicht wird.

### Ausführungsbeispiel

Die nachfolgenden Zeichnungen stellen einen Schnitt durch die erfindungsgemäße Vorrichtung 1 dar, wobei:
- Fig. 1 rein im Umluftbetrieb arbeitet.
- Fig. 2 eine Frischluft- oder Außenluftanschluss 15 aufweist, sodass eine Mischkammer 16 im Rohluftraum 13 der Vorrichtung 1 entsteht.
- Fig. 3 eine Frischluft- oder Außenluftanschluss 15 aufweist, sodass die Mischkammer 16 im Reinluftraum 14 der Vorrichtung 1 entsteht.

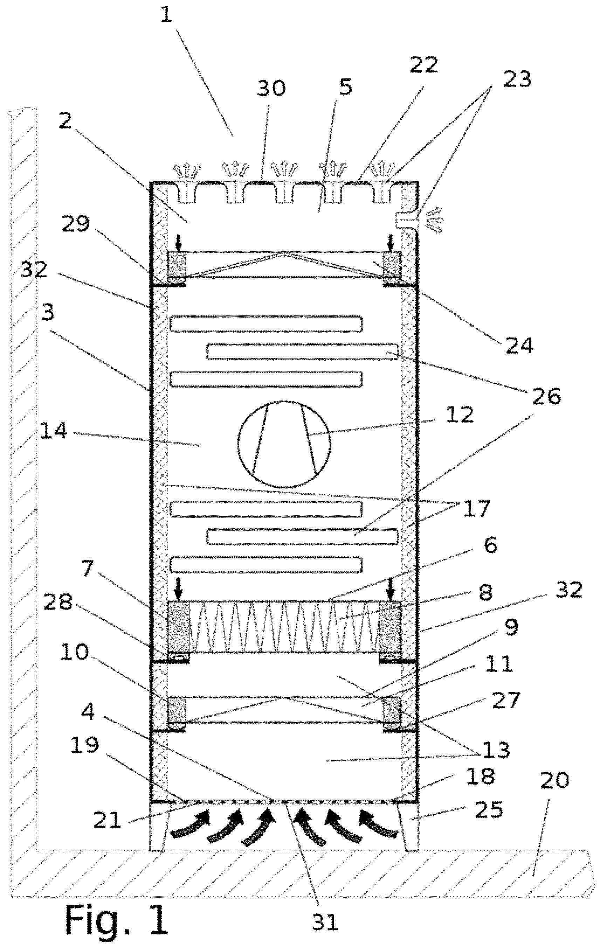

Die Vorrichtung 1 entsprechend der Fig. 1 weist ein vorzugsweise aus *Edelstahl,* sowie einer oberen Wandung 30, einer unteren Wandung 31 und seitlichen Wandungen 32, bestehendes, quaderförmiges Gehäuse 3 auf, das auf Bodenabstandshaltern 25, vorzugsweise Füßen oder Rollen steht und vom Fußboden 20 ausgehend aus einem Lufteintrittsquerschnitt 4, einer Vorfilterstufe 9, einer Hauptfilterstufe 6, schalldämmenden Elementen 26, einer Luftfördereinrichtung 12, einem Aktivkohlefilter 24 sowie einem Luftaustrittsquerschnitt 5 besteht.

Der Lufteintrittsquerschnitt 4 wird aus den Durchbrüchen 18 eines Lochblechs 19 an der Unterseite 21 des Gehäuses 3 gebildet.

Im Innenraum 2 des Gehäuses 3 ist oberhalb des Lufteintrittsquerschnitts 4 eine von den seitlichen Wandungen 32 des Gehäuses 3 horizontal nach innen vorkragende, umlaufende Sitzfläche 27 zum Aufnehmen einer angepassten Sitzfläche der Vorfilterstufe 9 angeordnet, die aus einem ersten Filterelement 10 mit einem ersten Filtermedium 11 besteht. Eine räumlich darüber angeordnete zweite horizontal ausgerichtete Sitzfläche 28 dient zum Aufnehmen einer angepassten Sitzfläche der Hauptfilterstufe 6 besteht aus einem zweiten Filterelement 7, vorzugsweise einem HEPA-Filter, mit einem zweiten Filtermedium 8. Zur Abdichtung des Reinluftraumes wird die Hauptfilterstufe 6 pressend mittels einer Kniehebeleinichtung auf der zugeordneten Sitzfläche 28 befestigt, sodass die Hauptfilterstufe 6 werkzeuglos ausgetauscht werden kann. Das zweite Filtermedium 8 und erste Filtermedium 11, sowie die zugehörigen Filterelemente 7,10 und die Flächen im Rohluftraum, die mit der Luft in diesem Raum in Kontakt stehen, sind mit einer antimikrobiell wirksamen Substanz beschichtet.

Die Luftfördereinrichtung 12 ist stromaufwärts und stromabwärts von plattenförmigen, schalldämmenden Elementen 26 begrenzt. Solche schalldämmenden Elemente 26 bestehen aus schallabsorbierendem Material, vorzugsweise Melaminharzplatten und sind umlaufend und der Strömungsrichtung abgewandten Seite in Blech, vorzugsweise aus Edelstahl, eingefasst.

Stromabwärts befindet sich eine weitere horizontale Sitzfläche 29 zum dichten Einbau eines Aktivkohlefilters 24.

Die Oberseite 22 sowie daran angrenzende Abschnitte der seitlichen Wandungen 32 des Gehäuses 3 weisen den Luftaustrittsquerschnitt 5 auf. Dieser besteht aus einer Mehrzahl von Luftdurchlässen 23, die in den Ausführungsbeispielen nach Fig. 1 bis Fig. 3 sowohl mit horizontaler als auch vertikaler Ausblasrichtung in dem Gehäuse 3 angeordnet sind.

Zur Schallabsorption ist das Gehäuse 3 an den Innenseiten seiner seitlichen Wandungen 32 vollflächig mit schallabsorbierendem Material 17, vorzugsweise Melaminharzplatten verkleidet.

Die Luftfördereinrichtung 12 fördert die Raumluft durch die Durchbrüche 18 des Lochblechs 19 des Lufteintrittsquerschnitts 4 in den Rohluftraum 13 und von dort durch das erste Filtermedium 11 der Vorfilterstufe 9 und das zweite Filtermedium 8 der Hauptfilterstufe 6 in den Reinluftraum 14. Die gefilterte Luft aus dem Reinluftraum 14 wird über einen Aktivkohlefilter 24 zum Luftaustrittsquerschnitt 5 gefördert und über die Luftdurchlässe 23 dem Raum zugeführt.

Die Oberflächen der im Rohluftraum 13 verbauten Komponenten sind mit einer antimikrobiell wirkenden Substanz versehen, sodass insbesondere Viren, z.B. auch Coronaviren, beim Kontakt mit den oben genannten Oberflächen inaktiviert werden. Damit sind speziell solche Oberflächen gemeint, die dem Rohluftraum 13 zugewandt sind.

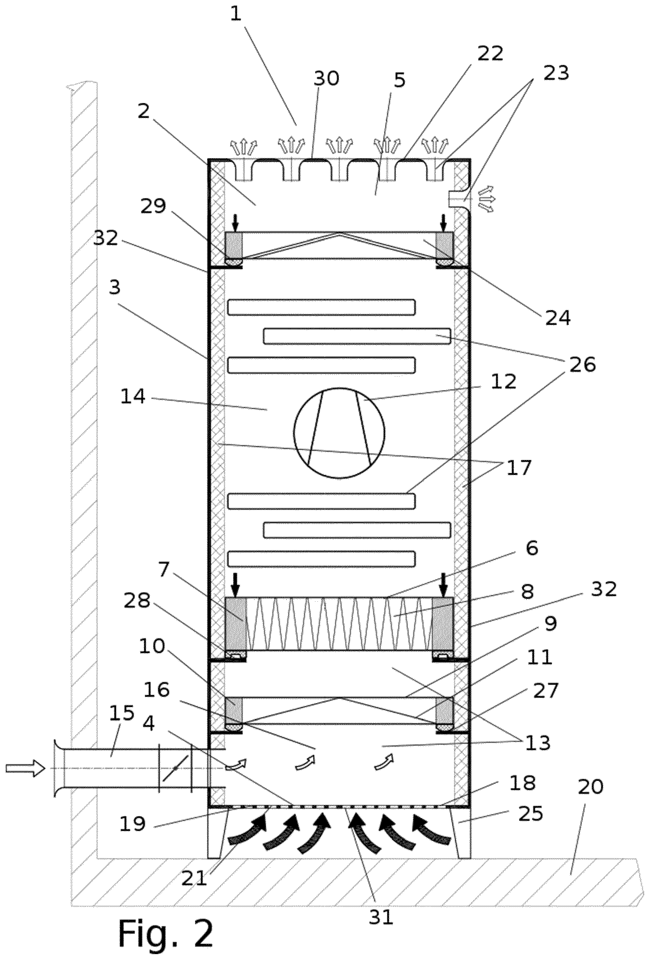

Eine weitere Ausführung abgebildet in Fig. 2 verfügt über einen zusätzlichen, unterhalb der Vorfilterstufe 9 angeordneten Frischluft- oder Außenluftanschluss 15, sodass Frischluft oder Außenluft mit der Raumluft in dem Rohluftraum 13 vermischt werden.

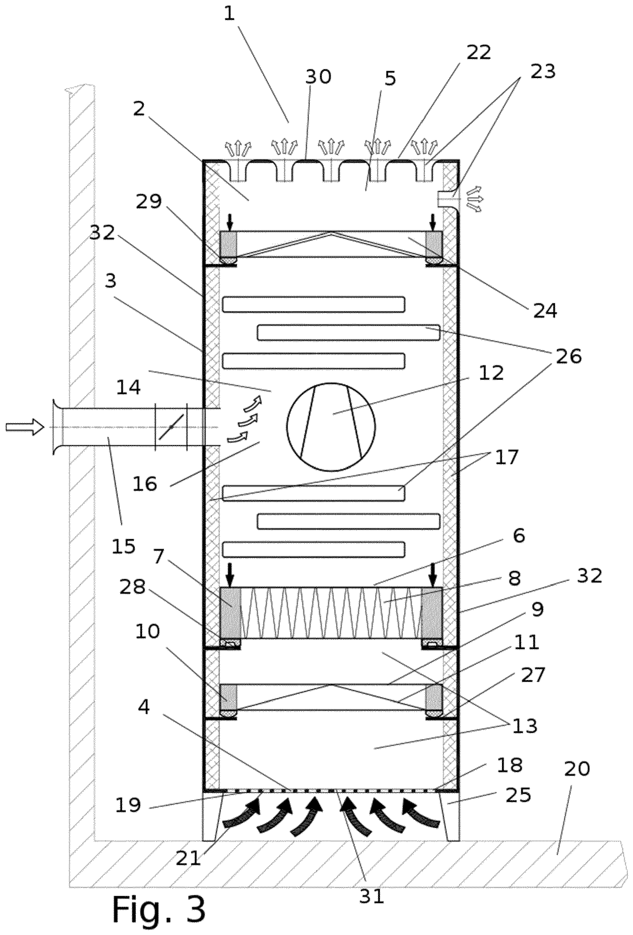

Eine weitere Ausführung abgebildet in Fig. 3 verfügt über einen zusätzlichen Frischluft- oder Außenluftanschluss 15, der oberhalb der Hauptfilterstufe 6 in das Gehäuse 3 mündet, sodass Frischluft oder Außenluft mit der Raumluft, ohne die Vor- 9 und Hauptfilterstufe 6 passieren zu müssen, in dem Reinluftraum 14 vermischt werden.

### Bezugszeichenliste:

- 1: Vorrichtung
- 2: Innenraum
- 3: Gehäuse
- 4: Lufteintrittsquerschnitt
- 5: Luftaustrittsquerschnitt
- 6: Hauptfilterstufe
- 7: Zweites Filterelement
- 8: Zweites Filtermedium
- 9: Vorfilterstufe
- 10: Erstes Filterelement
- 11: Erstes Filtermedium
- 12: Luftfördereinrichtung
- 13: Rohluftraum
- 14: Reinluftraum
- 15: Frischluft- oder Außenluftanschluss
- 16: Mischkammer
- 17: Akustische Dämmeigenschaften aufweisendes Material
- 18: Durchbruch
- 19: Lochblech
- 20: Fußboden
- 21: Unterseite des Gehäuses
- 22: Oberseite des Gehäuses
- 23: Luftdurchlass
- 24: Aktivkohlefilter
- 25: Bodenabstandshaltern
- 26: Schalldämmende Elemente
- 27: Sitzfläche Vorfilterstufe
- 28: Sitzfläche Hauptfilterstufe
- 29: Sitzfläche Aktivkohlefilter
- 30: Obere Wandung
- 31: Untere Wandung
- 32: Seitliche Wandung

## Patentansprüche

1. Vorrichtung (1) zur Filtration von Luft, umfassend
- ein einen Innenraum (2) umschließendes Gehäuse (3) mit einem Lufteintrittsquerschnitt (4) und einem Luftaustrittsquerschnitt (5),
- einer Vorfilterstufe (9) mit einem ersten Filterelement (10) mit einem ersten Filtermedium (11),
- eine Hauptfilterstufe (6) mit einem zweiten Filterelement (7) mit einem zweiten Filtermedium (8),
- eine Luftfördereinrichtung (12), mit der die Luft von dem Lufteintrittsquerschnitt (4) durch die Vorfilterstufe (9) und die Hauptfilterstufe (6) zu dem Luftaustrittsquerschnitt (5) förderbar ist,
wobei das zweite Filterelement (7) den Innenraum (2) in einen Rohluftraum (13), in dem sich die Vorfilterstufe (9) befindet, und einen Reinluftraum (14), in dem sich die Luftfördereinrichtung (12) befindet, unterteilt, **dadurch gekennzeichnet, dass** mit Luft in Berührung kommende Oberflächen, die den Rohluftraum (13) begrenzen, mit einer antimikrobiell wirksamen, insbesondere Viren inaktivierenden, Substanz versehen sind.

2. Vorrichtung (1) nach Anspruch 1, **dadurch gekennzeichnet,** durch einen Frischluft- oder Außenluftanschluss (15) und eine in den Reinluftraum (14) oder den Rohluftraum (13) angeordnete Mischkammer (16), in der Frisch- oder Außenluft mit der gefilterten Luft mischbar und sodann vorzugsweise mittels derselben Luftfördereinrichtung (12) zu dem Luftaustrittsquerschnitt (5) förderbar ist.

3. Vorrichtung (1) nach Anspruch 1 oder 2, **dadurch gekennzeichnet, dass** der Innenraum (2) des Gehäuses (3) mit schalldämmenden Material (17), insbesondere einem Schaumaterial oder gepressten Fasern bestehenden Material, isoliert ist, wobei die im Rohluftraum (13), angeordneten Material mit einer antimikrobiell wirkenden Substanz versehen, insbesondere damit beschichtet sind und zwar insbesondere auf ihrer dem Innenraum (2) zugewandten Seite.

4. Vorrichtung (1) nach dem vorhergehenden Anspruch, **dadurch gekennzeichnet, dass** das akustische Dämmeigenschaften aufweisende Material (17) plattenförmig ist und allein klemmend in dem Rohluftraum (13) in seiner eingebauten Funktion gehalten ist.

5. Vorrichtung (1) nach einem der vorhergehenden Ansprüche, **dadurch gekennzeichnet, dass** das zweite Filtermedium (8) und/oder das erste Filtermedium (11) durch Tauchen und/oder Besprühen und/oder sonstiges Auftragen eines fließfähigen Stoffes mit der antibakteriell wirksamen Beschichtung versehen.

6. Vorrichtung (1) nach einem der vorhergehenden Ansprüche, **dadurch gekennzeichnet, dass** die Wandungen des erste Filterelements (10) und/oder zweite Filterelements (7) mit einer antimikrobiell wirksamen Substanz versehen sind.

7. Vorrichtung (1) nach einem der vorhergehenden Ansprüche, **dadurch gekennzeichnet, dass** der Lufteintrittsquerschnitt (4) von Durchbrüchen (18) eines Lochblechs (19) gebildet wird, wobei eine dem Rohluftraum (13) zugewandte Innenseite des Lochblechs (19) mit einer antimikrobiell wirksamen Beschichtung versehen ist.

8. Vorrichtung (1) nach einem der vorhergehenden Ansprüche, **dadurch gekennzeichnet, dass** das Gehäuse (3) ungefähr quaderförmig und zur Aufstellung auf einem Fußboden (20) vorgesehen ist, wobei eine parallel zu dem Fußboden (20) verlaufende Unterseite (21) des Gehäuses (3) den Lufteintrittsquerschnitt (4) aufweist und eine parallel zu dem Fußboden (20) verlaufende und dem Lufteintrittsquerschnitt (4) gegenüberliegende Oberseite (22) des Gehäuses (3) den Luftaustrittsquerschnitt (5) aufweist.

9. Vorrichtung (1) nach einem der vorhergehenden Ansprüche, **dadurch gekennzeichnet, dass** eine Oberseite (22) des Gehäuses (3) und/ oder mindestens ein oberer Abschnitt mindestens einer seitlichen Wandung (32) mit einem Luftdurchlass (23), insbesondere einem Drall-Luftdurchlass und/ oder einem Luftverteilelement, versehen ist.

10. Vorrichtung (1) nach einem der vorhergehenden Ansprüche, **dadurch gekennzeichnet,** durch eine Differenzdruck-Messeinrichtung, mittels der ein Differenzdruck zwischen einer Eintrittsseite der Hauptfilterstufe (6) oder der Vorfilterstufe (9) und einer zugeordneten Austrittsseite messbar ist.
